# EUROPEAN PATENT SPECIFICATION

(11) **EP 1 466 178 B1**
(45) Date of publication and mention of the grant of the patent: **16.02.2011**
(21) Application number: 02787209.2
(22) Date of filing: 30.12.2002
(51) Int. Cl.: G01N 33/573, G01N 33/53, G01N 33/566, G01N 33/68, A61K 38/08, A61K 38/10, A61P 25/28, C12Q 1/02, C07K 7/06, C07K 7/08

(54) **SCREENING METHODS FOR SUPEROXIDE DISMUTASE (SOD)-INHIBITORS**
SCREENING-VERFAHREN FÜR INHIBITOREN VON SUPEROXID-DISMUTASE (SOD)
PROCEDES DE CRIBLAGE DES INHIBITEURS DE SUPEROXYDE-DISMUTASE (SOD)

(30) Priority: 27.12.2001 AU PR976401
(43) Date of publication of application: 13.10.2004
(73) Proprietor: Alzhyme Pty Ltd, Perth, Western Australia 6000 (AU)
(72) Inventor: MARTINS, Ralph, Maddington, Western Australia 6109 (AU)
(74) Representative: Sonn & Partner Patentanwälte
(86) International application number: PCT/AU2002/001754
(87) International publication number: WO 2003/056339

(56) References cited:
- WO-A-02/46221
- WO-A-95/08999
- WO-A-98/40071
- WO-A1-00/66181
- WO-A1-01/07442
- US-B1- 6 331 440
- US-B1- 6 365 414
- HILBICH E A C: "Substitutions of hydrophobic amino acids reduce the amyloidogenicity of Alzheimer's disease beta-A4 peptides" JOURNAL OF MOLECULAR BIOLOGY, LONDON, GB, no. 228, 1992, pages 460-473, XP002010983 ISSN: 0022-2836
- BAYER THOMAS A ET AL: "Dietary Cu stabilizes brain superoxide dismutase 1 activity and reduces amyloid Abeta production in APP23 transgenic mice" PROCEEDINGS OF THE NATIONAL ACADEMY OF SCIENCES OF USA, NATIONAL ACADEMY OF SCIENCE, WASHINGTON, DC, US, vol. 100, no. 24, 25 November 2003 (2003-11-25), pages 14187-14192, XP002339932 ISSN: 0027-8424
- JOBLING M.F. ET AL.: 'Copper and zinc binding modulates the agggregation and neurotoxic properties of the prion peptide PrP106-126' BIOCHEMISTRY vol. 40, June 2001, pages 8073 - 8084, XP002997338
- ATWOOD C.S. ET AL.: 'Characterization of copper interactions with Alzheimer amyloid beta peptides: Identification of an attomolar-affinity copper binding site on amyloid beta1-42' J. NEUROCHEM. vol. 75, no. 3, 2000, pages 1219 - 1233, XP002997339
- BUSH A.I.: 'Metal complexing agents as therapies for Alzheimer's disease' NEUROBIOLOGY OF AGING vol. 23, 2002, pages 1031 - 1038, XP002978709
- DATABASE GENBANK [Online] 06 September 2001 INOUE J.G. ET AL.: 'NADH dehydrogenase subunit 5 (Pantodon buchholzi)', XP002997355 Database accession no. (NP203724)
- DATABASE PROTEIN [Online] 23 March 2001 THEOLOGIS A. ET AL.: 'Hypothetical protein F11M15.16 (Arabidopsis thaliana)', XP002997395 Database accession no. (H96550)
- DATABASE PROTEIN [Online] 13 August 2001 TABATA S. ET AL.: 'Helicase-like protein (Arabidopsis thaliana)', XP002997396 Database accession no. (NP199293)

## Description

### Field of the Invention

The present invention relates to agents and methods for treating Alzheimer's disease and to methods for identifying agents for use in treating Alzheimer's disease. More particularly, the present invention relates to novel peptides and their use to treat Alzheimer's disease and methods for screening of peptides to identify peptides for use in treating Alzheimer's disease.

### Background

Alzheimer's disease (AD) is a progressive neurodegenerative disorder characterised pathologically by the deposition of amyloid plaques and neurofibrillary tangles, and neuronal degeneration, in the brains of affected individuals.

The major protein component of the amyloid deposits is a small 4 kDa peptide of 39-43 amino acids termed beta amyloid (ss-amyloid or Aβ). Numerous studies have suggested that Aβ accumulation and deposition may be critical to AD. The initial deposition of Aβ and growth of plaques has been suggested to occur via distinct processes. Aβ may either form higher oligomeric structures or remain in the monomeric form when it is deposited. In vitro studies have found that freshly solubilsed monomeric Aβ, at low concentrations, is not toxic to neurons in culture. However, after an aging period of several hours to days Aβ spontaneously aggregates in solution to form fibrilla entities that are highly neurotoxic. This suggests aggregation is a requirement for Aβ toxicity.

The AD brain has been shown to be under oxidative stress. Oxidative stress is a situation where there is an excess of oxygen free radicals or reactive oxygen species (ROS), which in turn damage surrounding tissue. Important ROS that damage the surrounding cellular components are the superoxide radical, hydrogen peroxide and the hydroxyl radical.

Superoxide dismutases (SOD) are metallo enzymes containing a redox-active transition metal (copper, iron or manganese) at the active site. SOD enzymes (SODs)-dismutate superoxide radicals by electron transfer between superoxide anions and the transition active metal. While low levels of SODs do provide some protection by detoxifying superoxide radicals thereby preventing superoxide mediated cell damage, high levels of these enzymes sensitises cells to oxidative stress by the overproduction of hydrogen peroxide.

There is data indicative of Aβ possessing SOD activity and that this activity is copper dependent. These findings have led to the development of copper cheating agents for treating AD. However, whilst copper chelators may inhibit the SOD activity of Aβ, they are non-specific and the chelation of copper may disrupt essential biochemical functions in the brain and result in undesirable side effects.

Other diseases such as type II diabetes, Scrapie and Transmissible Spongiform Encephalopathies such as Creutzfeldt Jacob disease (CJD), variant CJD, Gerstmann Strausler Schinkler syndrome and Bovine Spongiform Encephalopathy (BSE) have also been linked with SOD activity.

WO 98/40071 A describes a method for the identification of agents for the treatment of Alzheimer's disease, which interfere with the production of radical oxygen species.

Hilbich et al. (J. Mol. Biol. 228 (1992): 460-473) provides structural information on β-A4 peptides and derivates thereof relevant for the amyloidogenicity of Alzheimer's disease.

Bayer et al. (PNAS 100 (24) (2003): 14187-14192) discloses that stabilization of SOD 1 can reduce amyloid Aβ production.

According to the WO 02/46221 A2 a protein isoform, referred to ADPI-41 is provided which has been found to been differentially expressed in Alzheimer's disease.

WO 00/66181 A1 describes a method for the identification of an agent to be used in a treatment of Alzheimer's disease which inhibits hydrogen peroxide formation but does not inhibit SOD.

WO 01/07442 A1 discloses β-amyloid peptide inhibitors which bind to or destabilise the N-terminal loop of β-amyloid.

The US 6,365,414 discloses an assay for the detection of Aβ formation in biological fluids.

Peptides which block the amnestic effects of a peptide homologous to amyloid β protein are disclosed in the WO 95/08999 A.

The US 6,331,440 describes the identification of organic compounds capable of inhibiting the polymerisation of amyloid β peptide.

Jobling et al. (Biochemistry 40 (2001): 8073-8084) showed that neurotoxic aggregation of a prion peptide can be reduced by removing copper and zinc ions.

Atwood et al. (Neurochem. 75 (3) (2000): 1219-1233) disclose that copper and zinc ions bind to amyloid β which explains their enrichment in plaque pathology.

Bush (Neurobiology of Aging 23 (2002): 1031-1038) describes that metal complexing agents can be used for therapies for Alzheimer's disease. The metal binding compounds inhibit the generation of hydrogenperoxid by Aβ.

The present invention seeks to provide a screening method for identifying agents that more specifically inhibit the SOD activity and/or metal ion binding of causative agents such as Aβ and thus represent more attractive options for treatment of AD. At the very least, the present invention seeks to provide alternative agents to the agents currently used to treat this disease.

### Summary of the Invention

The present invention provides a method for isolating candidate peptides for the treatment of Alzheimer's disease, the method comprising the steps of:
(i) contacting a plurality of candidate peptides with Aβ and isolating the bound peptides; and
(ii) contacting the peptides isolated from step (i) with a second agent structurally related to Aβ but without SOD activity and isolating the unbound peptides, or
(ii) when Aβ's SOD activity is dependent on binding copper, contacting the peptides isolated from step (i) with a second agent that is structurally related to Aβ but is not adapted to binding copper and isolating the unbound peptides; or
(ii) when Aβ's toxicity is dependent on binding copper, contacting the peptides isolated from step (i) with a second agent that is adapted to bind peptides isolated from step (i) that do not specifically bind to the copper binding site of the Aβ and isolating the unbound peptides;
wherein the peptides isolated from step (ii) are candidate peptides for treatment of the disease.

A further aspect of the present invention is a method for isolating candidate peptides for the treatment of Alzheimer's disease, the method comprising the steps of:
(i) contacting a plurality of candidate peptides with a first agent that is adapted to bind peptides that specifically bind to the zinc binding site of Aβ and isolating the bound peptides; and
(ii) contacting the peptides isolated from step (i) with a second agent that binds peptides isolated from step (i) that do not specifically bind to the zinc binding site of Aβ and isolating the unbound peptides;
wherein the peptides isolated from step (ii) are candidate peptides for AD treatment.

The present invention also relates to peptides identified using the method of the invention and in particular to a peptide consisting of sequence selected from the group of sequences:
Met-Thr-Met-Pro-Thr-Met;
Pro-Leu-Pro-Gln-Met-Leu; and
Thr-Asn-Pro-Asn-Arg-Arg-Asn-Arg-Thr-Pro-Gln-Met-Leu-Lys-Arg;
or a functional variant thereof which is at least 80% identical to the recited sequence and which retains its ability to bind to a causative agent of Alzheimer's disease, said causative agent having SOD activity, and inhibit the causative agent's SOD and /or metal ion binding ability.

The present invention also relates to the peptides for use in treating Alzheimer's disease and for destabilizing multimeric forms of Aβ and for limiting or preventing the aggregation Aβ.

### Detailed Description of the Invention

### Screening Methods

The present invention provides a method for isolating candidate peptides for the treatment of Alzheimer's disease, the method comprising the steps of:
(i) contacting a plurality of candidate peptides with Aβ and isolating the bound peptides; and
(ii) contacting the peptides isolated from step (i) with a second agent structurally related to Aβ but without SOD activity and isolating the unbound peptides; or
(ii) when Aβ's SOD activity is dependent on binding copper, contacting the peptides isolated from step (i) with a second agent that is structurally related to Aβ but is not adapted to binding copper and isolating the unbound peptides; or
(ii) when Aβ's toxicity is dependent on binding copper, contacting the peptides isolated from step (i) with a second agent that is adapted to bind peptides isolated from step (i) that do not specifically bind to the copper binding site of the Aβ and isolating the unbound peptides;
wherein the peptides isolated from step (ii) are candidate peptides for treatment of the disease.

For the purposes of the present invention "structurally related" agents are agents that are related in terms of their amino acid sequence to the extent that the agents belong to the same class of molecules. For example, human and rat Aβ are structurally related for the purposes of the present invention. Preferably, structurally related agents have at least 75% amino acid sequence homology, more preferably at least 90% amino acid sequence homology and even more preferably at least 95-99% amino acid sequence homology.

It is also disclosed that the causative agent may be prion protein, Aβ or amylin. Furthermore, the disease or disorder may be selected from the group comprising: Scrapie and Transmissible Spongiform Encephalopathies such as Creutzfeldt Jacob disease (CJD), variant CJD, Gerstmann Strausler Schinkler syndrome and Bovine Spongiform Encephalopathy (BSE). According to the present invention, the disease or disorder is AD with its causative agent Aβ.

It is contemplated that the SOD activity of an agent may be dependent or otherwise related to the agent binding copper. Thus, disclosed is a method for isolating candidate peptides for the treatment of a disease or disorder caused by an agent whose SOD activity is dependent on binding copper, the method comprising the steps of:
(i) contacting a plurality of candidate peptides with a first agent with SOD activity that is adapted to specifically bind copper and isolating the bound peptides; and
(ii) contacting the peptides isolated from step (i) with a second agent that is structurally related to the first agent but is not adapted to bind copper and isolating the unbound peptides;
wherein the peptides isolated from step (ii) are candidate peptides for treatment of the disease.

The peptides isolated by screening based on disruption of SOD activity by interfering with copper binding may bind directly to the copper binding site of the first agent and thus directly block or otherwise interfere with copper binding. Alternatively, they may bind at a site other than the copper binding site and indirectly block copper binding by causing some conformational change that removes or otherwise affects the copper binding site to the extent that its ability to bind copper is at least diminished and preferably totally removed. It is also envisaged that peptides isolated using the screening method may disrupt SOD activity without necessarily preventing copper binding.

Also disclosed is a method for isolating candidate peptides for the treatment of a disease or disorder caused by an agent whose toxicity is dependent on binding copper, the method comprising the steps of:
(i) contacting a plurality of candidate peptides with a first agent that is adapted to bind peptides that specifically bind to the copper binding site of the causative agent and isolating the bound peptides; and
(ii) contacting the peptides isolated from step (i) with a second agent that is adapted to bind peptides isolated from step (i) that do not specifically bind to the copper binding site of the causative agent and isolating the unbound peptides;
wherein the peptides isolated from step (ii) are candidate peptides for treatment of the disease.

The first agent may be immobilised or in solution. The first agent may further comprise an isolation means that assists in isolating the peptides bound to the first agent. The isolation means may be any means that is adapted to selectively bind to another agent and includes peptides and antibodies or fragments thereof.
When the first agent is immobilised it may be immobilised by binding it to a surface via a biotin-streptavidin complex or some other alternative protein-protein complex.

The second agent may be immobilised or in solution. The second agent may further comprise an isolation means that assists in isolating the peptides bound to the second agent. The isolation means may be any means that is adapted to selectively bind to another agent and includes peptides, non-peptide molecules such as peptide mimetics and antibodies or fragments thereof.

When the second agent is immobilised it may be immobilised by binding it to a surface via a biotin-streptavidin complex or some other alternative protein-protein complex.

Preferably, the method steps (i) and (ii) are repeated to increase the selectivity of the method. When step (i) and (ii) are repeated, the amount of the first agent may be reduced in each repetition. In particular, the amount of the first agent may be reduced about 10 fold in each repetition. Steps (i) and (ii) may be repeated as many times as deemed necessary by a skilled person and preferably is repeated at least 2-3 times.

When steps (i) and (ii) are repeated, the peptides isolated in the first round may be amplified to provide a larger quantity for subsequent rounds. The amplification of the peptides may be achieved by any means apparent to those skilled in the art. Preferably, the peptides are amplified by growing them in a prokaryotic system such as bacteria.

### Hereunder specific mention is made to AD.

Thus, the present invention also provides a method for isolating candidate peptides for the treatment of AD the method comprising the steps of:
(i) contacting a plurality of candidate peptides with a first agent with SOD activity and isolating the bound peptides; and
(ii) contacting the peptides isolated from step (i) with a second agent structurally related to the first agent but without SOD activity and isolating the unbound peptides;
wherein the peptides isolated from step (ii) are candidate peptides for treatment of the disease.

The first agent is Aβ or a portion thereof containing a copper binding site and/or the portion of Aβ required for SOD activity. Preferably, the first agent is human Aβ such as human Aβ₁₋₄₂ or human Aβ₁₋₄₀. Alternatively, the first agent may be another mammalian Aβ that has SOD activity and/or includes a copper binding site such as rabbit, guinea pig, dog, monkey, cow, sheep or polar bear Aβ.

The second agent is structurally related to Aβ or a portion thereof that lacks SOD activity and/or a copper binding site. Preferably, the second agent is rat Aβ such as rat Aβ₁₋₄₂ or rat Aβ₁₋₄₀. Alternatively, the second agent may be another mammalian Aβ that lacks SOD activity and/or a copper binding site such as mouse Aβ.

The present invention also provides a method for isolating candidate peptides for the treatment of AD, the method comprising the steps of:
(i) contacting a plurality of candidate peptides with a first agent that binds peptides that specifically bind to the copper binding site of Aβ and isolating the bound peptides; and
(ii) contacting the peptides isolated from step (i) with a second agent that binds peptides isolated from step (i) that do not specifically bind to the copper binding site of Aβ and isolating the unbound peptides;
wherein the peptides isolated from step (ii) are candidate peptides for AD treatment.

The screening method of the present invention may also be used to identify peptides that are able to affect the zinc binding activity of a causative agent and thus control its aggregation. Thus, the present invention also provides a method for isolating candidate peptides for the treatment of AD, the method comprising the steps of:
(i) contacting a plurality of candidate peptides with a first agent that binds peptides that specifically bind to the zinc binding site of Aβ and isolating the bound peptides; and
(ii) contacting the peptides isolated from step (i) with a second agent that binds peptides isolated from step (i) that do not specifically bind to the zinc binding site of Aβ and isolating the unbound peptides;
wherein the peptides isolated from step (ii) are candidate peptides for AD treatment.

The agents used in the method of the present invention may be naturally occurring molecules. However, it will be appreciated that the agents could be non-naturally occurring molecules that have been produced to mimic one or more characteristics of the naturally occurring molecules that are important for the screen. For example, the agent may be a construct comprising or mimicking the portion of the naturally occurring molecule that is required for SOD activity and/or copper binding. These agents can be constructed based on an analysis of the structure of the naturally occurring molecule that defines the parts of the molecule that are involved in copper binding and/or SOD activity. Once these parts are identified peptide mimetics with one or more characteristics of the natural molecule can be designed and used in the screen instead of the naturally occurring molecule.

The method of the present invention may involve the use of phage display to select the peptides of interest. Alternatively, the method of the present invention may involve the use of other techniques that enable the convenient screening of candidate peptides such as the yeast two hybrid system.

The above screening methods relate to the identification of peptides with therapeutic uses. However, it will be appreciated that the methods may also be used to screen for non-peptidic compounds that also have therapeutic uses. These non-peptidic compounds include peptide mimetics with one or more characteristics of the peptides identified using the screening methods described herein and thus may be adapted to block or otherwise reduce the SOD and/or copper binding activity of a causative agent.

Thus, disclosed is a method for isolating candidate non-peptidic compounds for the treatment of a disease or disorder caused by an agent with SOD activity, the method comprising the steps of:
(i) contacting a plurality of candidate non-peptidic compounds with a first agent with SOD activity and being causative of the disease or disorder and isolating the bound compounds; and
(ii) contacting the compounds isolated from step (i) with a second agent structurally related to the first agent but without SOD activity and isolating the unbound compounds;
wherein the compounds isolated from step (ii) are candidates for treatment of the disease.

The screening method for non-peptidic compounds may be used to isolate candidates for the treatment of AD, the method comprising the steps of:
(i) contacting a plurality of candidate non-peptidic compounds with a first agent that is adapted to bind compounds that specifically bind to the copper binding site of Aβ and isolating the bound compounds; and
(ii) contacting the compounds isolated from step (i) with a second agent that is adapted to bind compounds isolated from step (i) that do not specifically bind to the copper binding site of Aβ and isolating the unbound compounds;
wherein the compounds isolated from step (ii) are candidates for AD treatment.

Preferably, the candidate non-peptidic compounds are provided in a database or library that can be conveniently screened. However, the candidate non-peptidic compounds may also be derived from other sources.

### Peptides

The present invention also provides peptides identified using the screening method of the invention and includes peptides that are adapted to bind to a causative agent such as Aβ and inhibit its SOD activity and/or its copper and/or zinc binding ability.

The peptides of the invention adapted to bind to Aβ include a peptide comprising a sequence selected from the group of sequences:
(i) Met-Thr-Met-Pro-Thr-Met;
(ii) Pro-Leu-Pro-Gln-Met-Leu; and
(iii) Thr-Asn-Pro-Asn-Arg-Arg-Asn-Arg-Thr-Pro-Gln-Met-Leu-Lys-Arg;
or a functional variant thereof.

Preferably, the peptides of the present invention totally remove SOD activity and/or prevent the causative agent binding copper although, it will be appreciated that peptides that decrease the SOD activity and/or copper binding activity are also useful. The peptides of the present invention may bind to the causative agent such that the copper binding site is no longer able to bind copper or is able to bind it to a lesser extent. In this regard, the peptide may bind at or near the copper binding site and physically prevent the binding of copper. When the peptide binds at or near the copper binding site of Aβ it preferably binds at or near amino acids 5-14 of Aβ, more preferably amino acids 8-14 of Aβ and even more preferably at or near amino acid 13 of Aβ, which is a histidine residue.

Alternatively, the peptide may bind to the causative agent at a site removed from the copper binding site and disrupt the conformation (or 3-D structure) of the copper binding site to reduce or totally remove its ability to bind copper and/or its SOD activity. When the peptide disrupts the conformation of the copper binding site of Aβ it preferably binds and disrupts the conformation of amino acids 5-14 of Aβ, more preferably amino acids 8-14 and even more preferably amino acid 13 of Aβ.

In another form of the invention, the peptides are adapted to bind Aβ in a fashion that does not prevent or reduce copper binding but still has therapeutic effect through at least reducing the SOD activity of the causative agent.

Functional variants also include peptides with modified or different amino acids sequences that still retain their ability to bind to the causative agent and inhibit its SOD activity and/or copper binding ability. These functional variants include peptides with deletions, insertions, inversions, repeats and/or type substitutions. Preferably, functional variants are at least 80% identical to the recited sequence, more preferably at least 90% identical and even more preferably at least 95% identical.

Functional variants also include peptides (i) in which one or more of the amino acid residues are substituted with a conserved or non-conserved amino acid residue such as synthetic, non-naturally occurring analogues and/or natural amino acid residues; or (ii) in which one or more of the amino acid residues includes a substituent group; or (iii) fused with another compound such as a compound to increase the peptide's half life (e.g. water-soluble polymers such as polyethylene glycol); or (iv) fused with additional amino acids such as an IgG Fc fusion region peptide, a leader or secretory sequence, or a sequence that is used to aid the purification or isolation of the peptide e.g. glutathione-S-transferase (GST), hexahistidine, GAL4 (DNA binding and/or transcriptional activation domains) and β-galactosidase. It may also be convenient to include a proteolytic cleavage site between the fusion peptide partner and the peptide sequence of interest to allow removal of the fusion sequence. Preferably the fusion sequence does not hinder the binding of the peptide to the causative agent to inhibit its SOD activity and/or copper binding ability.

Particular conserved substitutions involve the substitution of a charged amino acid with an alternative charged amino acid or a negatively charged or neutral amino acid. Preferably, the changes are minor and do not have a negative impact on the ability of the peptide to bind to the causative agent and inhibit its copper binding ability and/or its SOD activity. Other conservative substitutions for the purposes of the present invention are exemplified in the table hereunder. However, it will be appreciated that skilled persons may also determine further conservative substitutions not specifically listed.

| | |
|---|---|
| Aromatic | Phenylalanine |
| | Tryptophan |
| | Tyrosine |
| | Histidine |
| Hydrophobic | Leucine |
| | Isoleucine |
| | Valine |
| Small | Alanine |
| | Serine |
| | Threonine |
| | Methionine |
| | Glycine |
| Acidic | Aspartic acid |
| | Glutamic acid |
| Basic | Arginine |
| | Lysine |
| | Histidine |
| Polar | Glutamine |
| | Asparagine |

Amino acids that are essential for the binding of the peptide to the causative agent to inhibit its ability to bind copper and/or remove or decrease its SOD activity may be determined by one skilled in the art using techniques known in the art, such as alanine scanning. Structural analysis such as crystallisation, nuclear magnetic resonance or photoaffinity labelling may also be used when developing functional variants. Alternatively, synthetic peptides corresponding to candidate functional variants may be produced and their ability to bind to the causative agent and inhibit its ability to bind copper and/or remove SOD activity assessed *in vitro.*

It will be appreciated that the amino acids in the peptides of the present invention that are required for binding to the causative agent and/or to remove its SOD activity may be incorporated into larger peptides and still maintain their binding characteristics. Thus, the peptides may be of various sizes provided they maintain their ability to bind the causative agent and/or remove SOD activity. Preferably, the amino acids required for binding are a contiguous sequence of between about 5 and 20 amino acids and more preferably between about 6 and 15 amino acids. As indicated above this contiguous sequence may be incorporated into a larger peptide.

### Nucleotides

The present invention also provides polynucleotides encoding the peptides of the invention. It will be understood by a skilled person that numerous different polynucleotides can encode the same peptide as a result of the degeneracy of the genetic code. In addition, it is to be understood that skilled persons may, using routine techniques, make nucleotide substitutions that do not affect the peptide sequence encoded by the polynucleotides of the invention to reflect the codon usage of any particular host organism in which the polypeptides of the invention are to be expressed.

Polynucleotides of the invention may comprise DNA or RNA. They may be single-stranded or double-stranded. They may also be polynucleotides that include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of the present invention, it is to be understood that the polynucleotides described herein may be modified by any method available in the art. Such modifications may be carried out in order to enhance the *in vivo* activity or life span of polynucleotides of the invention.

Where the polynucleotide of the invention is double-stranded, both strands of the duplex, either individually or in combination, are encompassed by the present invention. Where the polynucleotide is single-stranded, it is to be understood that the complementary sequence of that polynucleotide is also included within the scope of the present invention.

### Therapeutic peptides

Peptides identified using the screening method of the present invention may be administered therapeutically to patients. It is preferred to use peptides that do not consist solely of naturally-occurring amino acids but which have been modified, for example to reduce immunogenicity, to increase circulatory half-life in the body of the patient, to enhance bioavailability and/or to enhance efficacy and/or specificity.

A number of approaches have been used to modify peptides for therapeutic application. One approach is to link the peptides or proteins to a variety of polymers, such as polyethylene glycol (PEG) and polypropylene glycol (PPG). Replacement of naturally occurring amino acids with a variety of uncoded or modified amino acids such as D-amino acids and N-methyl amino acids may also be used to modify peptides. Another approach is to use bifunctional crosslinkers, such as N-succinimidyl 3-(2 pyridyldithio) propionate, succinimidyl 6-[3-(2 pyridyldithio) propionamido] hexanoate, and sulfosuccinimidyl 6-[3-(2 pyridyldithio) propionamido]hexanoate.

It may be desirable to use derivatives of the peptides of the invention that are conformationally constrained. Conformational constraint refers to the stability and preferred conformation of the three-dimensional shape assumed by a peptide. Conformational constraints include local constraints, involving restricting the conformational mobility of a single residue in a peptide; regional constraints, involving restricting the conformational mobility of a group of residues, which residues may form some secondary structural unit; and global constraints, involving the entire peptide structure.

The active conformation of the peptide may be stabilized by a covalent modification, such as cyclization or by incorporation of gamma-lactam or other types of bridges. For example, side chains can be cyclized to the backbone so as create a L-gamma-lactam moiety on each side of the interaction site. Cyclization also can be achieved, for example, by formation of cystine bridges, coupling of amino and carboxy terminal groups of respective terminal amino acids, or coupling of the amino group of a Lys residue or a related homolog with a carboxy group of Asp, Glu or a related homolog. Coupling of the alpha-amino group of a polypeptide with the epsilon-amino group of a lysine residue, using iodoacetic anhydride, can be also undertaken.

Another approach is to include a metal-ion complexing backbone in the peptide structure. Typically, the preferred metal-peptide backbone is based on the requisite number of particular coordinating groups required by the coordination sphere of a given complexing metal ion. In general, most of the metal ions that may prove useful have a coordination number of four to six. The nature of the coordinating groups in the peptide chain includes nitrogen atoms with amine, amide, imidazole, or guanidino functionalities; sulfur atoms of thiols or disulfides; and oxygen atoms of hydroxy, phenolic, carbonyl, or carboxyl functionalities. In addition, the peptide chain or individual amino acids can be chemically altered to include a coordinating group, such as for example oxime, hydrazino, sulfhydryl, phosphate, cyano, pyridino, piperidino, or morpholino. The peptide construct can be either linear or cyclic, however a linear construct is typically preferred. One example of a small linear peptide is Gly-Gly-Gly-Gly that has four nitrogens (an N₄ complexation system) in the backbone that can complex to a metal ion with a coordination number of four.

A further technique for improving the properties of therapeutic peptides is to use non-peptide peptidomimetics. A wide variety of useful techniques may be used to elucidating the precise structure of a peptide. These techniques include amino acid sequencing, x-ray crystallography, mass spectroscopy, nuclear magnetic resonance spectroscopy, computer-assisted molecular modelling, peptide mapping, and combinations thereof. Structural analysis of a peptide generally provides a large body of data that comprise the amino acid sequence of the peptide as well as the three-dimensional positioning of its atomic components. From this information, non-peptide peptidomimetics may be designed that have the required chemical functionalities for therapeutic activity but are more stable, for example less susceptible to biological degradation.

Thus, the present invention also provides for the use of a peptide identified using the screening method described herein for designing a mimetic thereof such as a non-peptide peptidomimetic.

### Other methods

The peptides of the present invention are adapted to bind to the causative agents of disease and remove their SOD activity and/or copper binding ability. Thus, the present invention also provides a method for reducing or removing SOD activity or inhibiting the copper binding ability of a causative agent, the method comprising contacting the causative agent with a peptide identified using the screen of the present invention such that the peptide binds to the causative agent in a fashion that at least reduces its SOD and/or copper binding activity. Copper is involved in stabilizing multimeric forms of Aβ. Thus, the present invention also provides a method for destabilizing multimeric forms of Aβ, the method comprising contacting the multimeric Aβ with a peptide of the present invention such that the peptide binds to the multimeric Aβ in a fashion that destabilizes the multimeric Aβ.

The present invention also provides a method for limiting or preventing the aggregation of Aβ, the method comprising contacting the Aβ with a peptide of the present invention such that the peptide binds to the Aβ and prevents or limits its aggregation.

It is disclosed that the causative agents include prion protein, Aβ and amylin. Also contemplated is treating a disease or disorder selected from the group comprising type 11 diabetes, AD, Scrapie and Transmissible Spongiform Encephalopathies such as Creutzfeldt Jacob disease (CJD), variant CJD, Gerstmann Strausler Schinkler syndrome and Bovine Spongiform Encephalopathy (BSE) comprising the step of administering an effective amount of a peptide identified using the screening method.

### Administration

Peptides of the invention may be combined with various components to produce compositions of the invention. Preferably the compositions are combined with a pharmaceutically acceptable carrier or diluent to produce a pharmaceutical composition (which may be for human or animal use). Suitable carriers and diluents include isotonic saline solutions, for example phosphate-buffered saline.

The composition of the invention may be administered by direct injection. The composition may be formulated for parenteral, intramuscular, intravenous, subcutaneous, intraocular, oral or transdermal administration.

The routes of administration described herein are intended only as a guide since a skilled practitioner will be able to determine readily the optimum route of administration and dosage for any particular patient.

The present invention will now be described with reference to the examples that describe one preferred embodiment of the invention. The description of the examples is in no way limiting on the scope of the present invention described elsewhere herein.

### Examples

### Example 1 - Phage Display

The two phage libraries (fUSE 5/6-mer and fUSE 5/15-mer peptide inserts, can be obtained from Professor George P Smith from the University of Missouri, Columbia, MO, USA other suitable phage libraries can be purchased from commercial sources such as New England Biolabs Inc. Bio/Can Scientific Inc, DGI BioTechnologies LLC. NoBiTec, Syncomm Corp, Invitrogen and Stratagene) were each selected with both human Aβ40 and human Aβ42 separately. The eluted selected phage then were dialysed before being subtracted with rat Aβ42 to give unbound fractions of phage. This sub-population therefore contains those peptides, which recognise human Aβ and have had subtracted, those which also recognise rat Aβ.

Four rounds of this selection/subtraction protocol are carried out, reducing the quantity of the HAβ by "10-fold" each time to encourage selection of the phage clones with the greatest affinity for the target molecule. After four rounds of selection/subtraction DNA is extracted and sequenced to determine level of consensus of sequences. A further three rounds of selection/subtraction are carried out to ensure sufficient duplication of sequences.

### Methodology

First Round panning pre-reacts the streptavidin with ligate (Aβ) before adding the phage.
ie. PLATE + LIGATE → PLATE-LIGATE + PHAGE

| | |
|---|---|
| 1. | Buffer preparation. |
| 2. | Biotinylation of ligate (Aβ) [human Aβ40 peptide (HAβ40) and human Aβ42 peptide (HAβ42). |
| 3. | Coat a 6 well sterile plate with Streptavidin (SA) overnight (O/N) at room temperature (RT) with gentle rocking. |
| 4. | Block SA coated plate with buffer A for 1 hr at 37°C with gentle rocking. |
| 5. | Add biotinylated HAβ40 and HAβ42 to SA coated plates and incubate at 37°C for 2 hrs with gentle rocking. |
| 6. | Add 4 µl of biotin to block remaining binding sites and incubate for a further 1 hr at 37°C. |
| 7. | Wash plate with buffer B, then add 0.4 ml of buffer B to 10 µl of the 6 mer and 15 mer phage libraries. Add this to the respective well of the SA/biotin plate and incubate at 37°C for 4 hrs. |
| 8. | Discard unbound phage, wash 10 times with buffer B and elute with 0.4 ml of buffer C. Collect eluate into eppendorf tube and add 75 µl of buffer D. |
| 9. | Dialyse eluates in buffer E with gentle agitation O/N at 4°C. |
| 10. | Biotinylate rat Aβ42 peptide (RAβ42) and incubate biotinylated product with 100µl of dialysed eluate O/N at 37°C with constant shaking |
| 11. | Coat a 6 well sterile plate with Streptavidin (SA) overnight (O/N) at room temperature (RT) with gentle rocking. |
| 12. | Repeat step 4. |
| 13. | Wash SA plate with buffer B, then add 0.4 ml of buffer B to eluate + biotinylated RAβ42 bound libraries. Add this to the respective well of the SA plate and incubate for 0.5 hr at 37°C then collect the unbound phage and store at 4°C. |
| 14. | Wash bottles, prepare buffers/broths and agars, autoclave and pour plates for growing K91 Kan E.coli without phage). |

| **Amplification for further rounds** | |
|---|---|
| 15. | Plate out E.coli for single colonies on TY+Kan agar plates for O/N growth at 37°C. |
| 16. | Pick a single colony and inoculate 1ml of TY broth + Kan. Culture for 5 hrs at 37°C |
| 17. | Mix 100 µl of unbound phage with 100 µl of cultured E.coli. and incubate at RT for 20 min, then transfer to tube containing 30 ml of TY +0.2 µg/ml Tet. Shake for 45 min at 37°C. Then add 30 µl of 20 mg/ml Tet and shake for 37°C O/N. |
| 18. | Centrifuge tubes for 45 min at 2,500 rpm at RT. Transfer supernatant to a new set of labelled tubes. Add 4.5 ml of buffer F to get rid of the bacteria, mix and incubate at 4°C O/N. |
| 19. | Centrifuge tubes for 45 min at 2,500 rpm at RT. Discard supernatant (pour off) and leave tubes inverted on paper towels for up to 1 hr. Add TBS buffer to each tube, mix phage thoroughly and transfer to labelled eppendorf tube. Centrifuge tube at 10,000 rpm for 3 min. Transfer the supernatant to a freshly labelled eppendorf tube. Add 0.15 ml of buffer F and leave at 4°C O/N. |
| 20. | Centrifuge tube at 10,000 rpm for 10 min. Discard supernatant. Resuspend pellet in 0.2 ml of buffer G and store at 4°C. |

The second and subsequent rounds of panning are then carried out. For these rounds the phage is pre-reacted with ligate (Aβ) before adding it to the streptavidin coated plate.
ie. PHAGE + LIGATE → PHAGE-LIGATE + PLATE For round Two Round two and subsequent panning rounds:

| | |
|---|---|
| 1. | Buffer preparation. |
| 2. | Biotinylation of ligate (Aβ) [human Aβ40 peptide (HAβ40) and human Aβ42 peptide (HAβ42). |
| 3. | Incubate biotinylated HAβ40 and HAβ42 peptides with amplified phage (there are four samples ie. 40/6mer, 40/15mer, 42/6mer and 42/15mer) O/N at 37°C. |
| 4. | Coat a 6 well sterile plate with Streptavidin (SA) overnight (O/N) at room temperature (RT) with gentle rocking. |
| 5. | Block SA coated plate with buffer A for 1 hr at 37°C with gentle rocking. |
| 6. | Wash plate with buffer B, then add 0.4 ml of buffer B to the four amplified phage samples. Add samples to the respective well of the SA plate and incubate at 37°C for 0.5 hr. |
| 7. | Discard unbound phage, wash 10 times with buffer B and elute with 0.4 ml of buffer C. Collect eluate into eppendorf tube and add 75 µl of buffer D. |
| 8. | Dialyse eluates in buffer E with gentle agitation O/N at 4°C. |
| 9. | Biotinylate rat Aβ42 peptide (RAβ42) and incubate biotinylated product with 100µl of dialysed eluate O/N at 37°C with constant shaking |
| 10. | Coat a 6 well sterile plate with Streptavidin (SA) overnight (O/N) at room temperature (RT) with gentle rocking. |
| 11. | Repeat step 4. |
| 12. | Wash SA plate with buffer B, then add 0.4 ml of buffer B to eluate + biotinylated RAβ42 bound libraries. Add this to the respective well of the SA plate and incubate for 0.5 hr at 37°C then collect the unbound phage and store at 4°C. |
| 13. | Wash bottles, prepare buffers/broths and agars, autoclave and pour plates for growing K91 Kan E.coli (with and without phage). |
| | **For Amplificaion after Round Two and Round Three: See Round One Amplification for further rounds.** |
| | **For Amplification of phage for Sequencing (from Round FOUR onwards):** |
| 14. | Prepare plates and plate out E.coli for single colonies on TY+Kan agar plate for O/N growth at 37°C. |
| 15. | Pick a single colony and inoculate 1 ml of TY broth + Kan. Culture for 5 hrs at 37°C. |
| 16. | Prepare serial dilutions (10⁻¹,10⁻²,10⁻³,10⁻⁴,10⁻⁵) of unbound phage. |
| 17. | Mix 20 µl of neat and diluted phage with 20 µl of cultured E.coli. and incubate at 37°C for 10 min, then at RT for 15 min. Add 1.0 ml of fresh TY broth to each sample and shake for 30 min at 37°C. Plate out 0.2 ml of each sample onto a separate TY+Kan+Tet agar plate for O/N growth at 37°C. |
| 18. | Select plate with an optimum number of about 20 colonies. Label colonies (A-T). For each Aβ form for either the 6 mer or 15 mer library, therefore n=4, will have 20 colonies, therefore 20x4=total of 80 colonies. Transfer each colony into its respective labelled tube containing 30 ml of Terrific broth+Kan+Tet (prepare fresh on the day of use). Incubate with shaking O/N at 37°C. |
| 19. | Centrifuge tubes for 45 min at 2,500 rpm at RT. Transfer supernatant to a new set of labelled tubes. Add 4.5 ml of buffer F to get rid of the bacteria, mix and incubate at 4°C O/N. |
| 20. | Centrifuge tubes for 45 min at 2,500 rpm at RT. Discard supernatant (pour off) and leave tubes inverted on paper towels for up to 1 hr. Add TBS buffer to each tube, mix phage thoroughly and transfer to labelled eppendorf tube. Centrifuge tube at 10,000 rpm for 3 min. Transfer the supernatant to a freshly labelled eppendorf tube. Add 0.15 ml of buffer F and leave at 4°C O/N. |
| 21. | Centrifuge tube at 10,000 rpm for 10 min. Discard supernatant. Resuspend pellet in 0.2 ml of buffer G. Extract DNA from phage using M13 kit for first 20 samples. |
| 22. | Finish off DNA extraction from remaining 60 samples. |
| 23. | Measure Absorbance of samples. Determine concentrations and prepare aliquots for sequencing. |
| 24. | Sequencing of DNA. This requires PCR, purify product by ethanol precipitation and run on ABI 310 sequencer. NOTE: THIS IS ONLY FOR ROUNDS 4 TO 7. |
| 25. | Data analysis of all sequences obtained. |

Three candidate peptides were identified when HAβ₁₋₄₂ was used to isolate a sub-population from a whole phage library and then RAβ₁₋₄₂, in excess, was used to 'mop-up' the phage clones that recognise common sites between the two Aβ molecules. This left behind in the unbound fraction of the applied phage, only those phage clones that specifically recognise those characteristics of HAβ₁₋₄₂ (in the copper binding site) that are unique to this human peptide and which confer on the molecule its SOD activity.

Sequences of three candidate Aβ binding peptides isolated using the above protocol are:

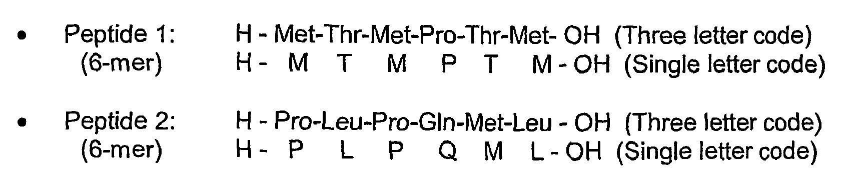

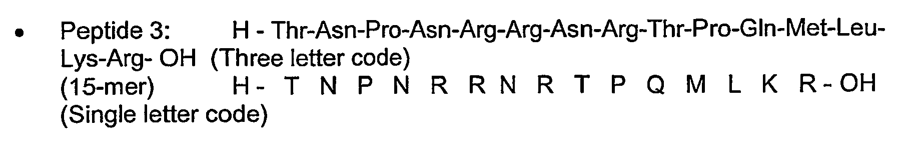

Throughout the specification, unless the context requires otherwise, the word "comprise" or variations such as "comprises" or "comprising", will be understood to imply the inclusion of a stated integer or group of integers but not the exclusion of any other integer or group of integers.

## Claims

1. A method for isolating candidate peptides for the treatment of Alzheimer's disease, the method comprising the steps of:
(i) contacting a plurality of candidate peptides with Aβ and isolating the bound peptides, and
(ii) contacting the peptides isolated from step (i) with a second agent structurally related to Aβ but without SOD activity and isolating the unbound peptides; or
(ii) when Aβ's SOD activity is dependent on binding copper, contacting the peptides isolated from step (i) with a second agent that is structurally related to Aβ but is not adapted to binding copper and isolating the unbound peptides; or
(ii) when Aβ's toxicity is dependent on binding copper, contacting the peptides isolated from step (i) with a second agent that is adapted to bind peptides isolated from step (i) that do not specifically bind to the copper binding site of the Aβ and isolating the unbound peptides;
wherein the peptides isolated from step (ii) are candidate peptides for treatment of the disease.

2. The method according to any one of claims 1 wherein the method steps (i) and (ii) are repeated to increase the selectivity of the method.

3. The method according to claim 1 wherein the Aβ contains a copper binding site and/or SOD activity or a portion thereof with SOD activity and/or a copper binding site.

4. The method according to claim 3 wherein the Aβ is human Aβ.

5. The method according to claim 3 wherein the Aβ is a non-human mammalian Aβ.

6. The method according to any one of claims 3 to 5 wherein the second agent is Aβ that lacks SOD activity and/or a copper binding site or a portion thereof that lacks SOD activity and/or a copper binding site.

7. The method according to claim 6 wherein the second agent is rat Aβ or mouse Aβ.

8. The method according to claim 1 for isolating candidate peptides for the treatment of Alzheimer's disease, the method comprising the steps of:
(i) contacting a plurality of candidate peptides with Aβ and isolating the bound peptides; and
(ii) contacting the peptides isolated from step (i) with a second agent that is adapted to bind peptides isolated from step (i) that do not specifically bind to the copper binding site of Aβ and isolating the unbound peptides;
wherein the peptides isolated from step (ii) are candidate peptides for Alzheimer's disease treatment.

9. A peptide consisting of a sequence selected from the group of sequences:
(i) Met-Thr-Met-Pro-Thr-Met;
(ii) Pro-Leu-Pro-Gln-Met-Leu; and
(iii) Thr-Asn-Pro-Asn-Arg-Arg-Asn-Arg-Thr-Pro-Gln-Met-Leu-Lys-Arg;
or a functional variant thereof which is at least 80% identical to the recited sequence and which retains its ability to bind to a causative agent of Alzheimer's disease, said causative agent having SOD activity, and inhibit the causative agent's SOD and/or metal ion binding ability.

10. The peptide according to claim 9 that binds at or near a copper binding site of Aβ and physically prevents the binding of copper.

11. The peptide according to claim 9 wherein the peptide binds at or near amino acids 5 to 14 of human Aβ, or amino acids 8 to 14 of human Aβ, or amino acid 13 of human Aβ.

12. The peptide according to claim 9 that binds to Aβ and disrupts the conformation (or 3-D structure) of the copper binding site to reduce or totally remove its ability to bind copper and/or its SOD activity.

13. The peptide according to claim 12 that binds and disrupts the conformation of amino acids 5 to 14 of human Aβ, or amino acids 8 to 14 of human Aβ, or amino acid 13 of human Aβ.

14. The peptide according to claim 9 that binds to Aβ and reduces or totally removes its SOD activity whilst still allowing the Aβ to bind copper.

15. A polynucleotide encoding any one of the peptides of claims 9 to 14.

16. A peptide according to any one of claims 9 to 14 for use in a therapeutic method in the treatment of Alzheimer's disease, preferably for reducing or removing SOD activity or inhibiting the copper binding ability of a causative agent of Alzheimer's disease, the method comprising contacting the causative agent with said peptide such that the peptide binds to the causative agent in a fashion that at least reduces its SOD and/or copper binding activity, or for destabilizing multimeric forms of Aβ, the method comprising contacting the multimeric Aβ with said peptide such that the peptide binds to the multimeric Aβ in a fashion that destabilizes the multimeric Aβ, or for limiting or preventing the aggregation of Aβ, the method comprising contacting the Aβ with said peptide such that the peptide binds to the Aβ and prevents or limits its aggregation.

17. A method for isolating candidate peptides for the treatment of Alzehimer's disease, the method comprising the steps of:
(i) contacting a plurality of candidate peptides with a first agent that binds peptides that specifically bind to the zinc binding site of Aβ and isolating the bound peptides; and
(ii) contacting the peptides isolated from step (i) with a second agent that binds peptides isolated from step (i) that do not specifically bind to the zinc binding site of Aβ and isolating the unbound peptides;
wherein the peptides isolated from step (ii) are candidate peptides for Alzheimer's disease treatment.

## Patentansprüche

1. Verfahren zum Isolieren von Peptiden, die für die Behandlung der Alzheimer-Krankheit in Frage kommen, wobei das Verfahren die folgenden Schritte umfasst:
(i) in Kontakt Bringen einer Vielzahl von in Frage kommenden Peptiden mit Aβ und Isolieren der gebundenen Peptide, und
(ii) in Kontakt Bringen der isolierten Peptide aus Schritt (i) mit einem zweiten Agens, das strukturell mit Aβ verwandt ist, aber keine SOD-Aktivität aufweist, und Isolieren der ungebundenen Peptide; oder
(ii) wenn die SOD-Aktivität des Aβ von einer Bindung von Kupfer abhängt, in Kontakt Bringen der isolierten Peptide aus Schritt (i) mit einem zweiten Agens, das strukturell mit Aβ verwandt ist, aber kein Kupfer binden kann, und Isolieren der ungebundenen Peptide; oder
(ii) wenn die Toxizität von Aβ von einer Bindung von Kupfer abhängt, in Kontakt Bringen der isolierten Peptide aus Schritt (i) mit einem zweiten Agens, das isolierte Peptide aus Schritt (i) binden kann, die sich nicht spezifisch an die Kupferbindungsstelle des Aβ binden, und Isolieren der ungebundenen Peptide;
wobei die isolierten Peptide aus Schritt (ii) Peptide sind, die für die Behandlung der Krankheit in Frage kommen.

2. Verfahren nach Anspruch 1, wobei die Verfahrensschritte (i) und (ii) wiederholt werden, um die Selektivität des Verfahrens zu erhöhen.

3. Verfahren nach Anspruch 1, wobei das Aβ eine Kupferbindungsstelle und/oder SOD-Aktivität oder einen Teil davon mit SOD-Aktivität und/oder einer Kupferbindungsstelle enthält.

4. Verfahren nach Anspruch 3, wobei das Aβ humanes Aβ ist.

5. Verfahren nach Anspruch 3, wobei das Aβ nicht-humanes Säuger-Aβ ist.

6. Verfahren nach einem der Ansprüche 3 bis 5, wobei das zweite Agens Aβ ist, das keine SOD-Aktivität und/oder Kupferbindungsstelle aufweist, oder ein Teil davon ist, der keine SOD-Aktivität und/oder Kupferbindungsstelle aufweist.

7. Verfahren nach Anspruch 6, wobei das zweite Agens Ratten-Aβ oder Mäuse-Aβ ist.

8. Verfahren nach Anspruch 1 zum Isolieren von Peptiden, die für die Behandlung von Alzheimer-Krankheit in Frage kommen, wobei das Verfahren die folgenden Schritte umfasst:
(i) in Kontakt Bringen einer Vielzahl von in Frage kommenden Peptiden mit Aβ, und Isolieren der gebundenen Peptide; und
(ii) in Kontakt Bringen der isolierten Peptide aus Schritt (i) mit einem zweiten Agens, das isolierte Peptide aus Schritt (i) binden kann, die sich nicht spezifisch an die Kupferbindungsstelle von Aβ binden, und Isolieren der ungebundenen Peptide;
wobei die isolierten Peptide aus Schritt (ii) Peptide sind, die für die Behandlung der Alzheimer-Krankheit in Frage kommen.

9. Peptid, das aus einer Sequenz besteht, die ausgewählt ist aus der Gruppe der Sequenzen:
(i) Met-Thr-Met-Pro-Thr-Met;
(ii) Pro-Leu-Pro-Gln-Met-Leu; und (iii) Thr-Asn-Pro-Asn-Arg-Arg-Asn-Arg-Thr-Pro-Gln-Met-Leu-Lys-Arg;
oder einer funktionalen Variante davon, die zu mindestens 80% identisch ist mit der genannten Sequenz, und das seine Fähigkeit, einen Verursacher der Alzheimer-Krankheit zu binden, wobei der Verursacher eine SOD-Aktivität aufweist, behalten hat, ebenso wie seine Fähigkeit, die SOD- und/oder Metallionen-Bindungsfähigkeit des Verursachers zu hemmen.

10. Peptid nach Anspruch 9, das sich an eine Kupferbindungsstelle von Aβ oder eine Stelle in deren Nähe bindet und physikalisch die Bindung von Kupfer verhindert.

11. Peptid nach Anspruch 9, wobei das Peptid an oder nahe an Aminosäuren 5 bis 14 des humanen Aβ oder Aminosäuren 8 bis 14 des humanen Aβ oder die Aminosäure 13 des humanen Aβ bindet.

12. Peptid nach Anspruch 9, das an Aβ bindet und die Konformation (oder 3-D-Struktur) der Kupferbindungsstelle unterbricht, wodurch dessen Kupferbindungsfähigkeit und/oder SOD-Aktivität herabgesetzt und vollständig aufgehoben wird.

13. Peptid nach Anspruch 12, das an die Aminosäuren 5 bis 14 von humanem Aβ oder die Aminosäuren 8 bis 14 von humanem Aβ oder an die Aminosäure 13 von humanem Aβ bindet und deren Konformation unterbricht.

14. Peptid nach Anspruch 9, das sich an Aβ bindet und dessen SOD-Aktivität herabsetzt oder vollständig aufhebt, aber immer noch zulässt, dass Aβ Kupfer bindet.

15. Polynukleotid, das eines der Peptide nach den Ansprüchen 9 bis 14 kodiert.

16. Peptid nach einem der Ansprüche 9 bis 14 zur Verwendung in einem therapeutischen Verfahren für die Behandlung der Alzheimer-Krankheit, vorzugsweise zur Herabsetzung oder Aufhebung der SOD-Aktivität oder zur Hemmung der Kupferbindungsfähigkeit eines Verursachers der Alzheimer-Krankheit, wobei das Verfahren das in Kontakt Bringen des Verursachers mit dem Peptid umfasst, damit das Peptid auf eine Weise an den Verursacher bindet, die dessen SOD- und/oder Kupferbindungs-Aktivität zumindest verringert, oder zur Destabilisierung multimerer Formen von Aβ, wobei das Verfahren das in Kontakt Bringen des multimeren Aβ mit dem Peptid umfasst, damit das Peptid auf eine Weise an das multimere Aβ bindet, die das multimere Aβ destabilisiert, oder zur Beschränkung oder Verhinderung der Aggregation von Aβ, wobei das Verfahren das in Kontakt Bringen des Aβ mit dem Peptid umfasst, damit das Peptid an das Aβ bindet und dessen Aggregation verhindert oder beschränkt.

17. Verfahren zum Isolieren von Peptiden, die für die Behandlung der Alzheimer-Krankheit in Frage kommen, wobei das Verfahren die folgenden Schritte umfasst:
(i) in Kontakt Bringen einer Vielzahl von in Frage kommenden Peptiden mit einem ersten Agens, das Peptide bindet, die sich spezifisch an die Zinkbindungsstelle von Aβ binden, und Isolieren der gebundenen Peptide; und
(ii) in Kontakt Bringen der isolierten Peptide aus Schritt (i) mit einem zweiten Agens, das isolierte Peptide aus Schritt (i) bindet, die sich nicht spezifisch an die Zinkbindungsstelle von Aβ binden, und Isolieren der ungebundenen Peptide;
wobei die isolierten Peptide aus Schritt (i) Peptide sind, die für die Behandlung der Alzheimer-Krankheit in Frage kommen.

## Revendications

1. Procédé pour isoler des peptides candidats pour le traitement de la maladie d'Alzheimer, le procédé comprenant les étapes consistant à :
(i) mettre en contact une pluralité de peptides candidats avec une Aβ et isoler les peptides liés, et
(ii) mettre en contact les peptides isolés à l'étape (i) avec un deuxième agent structurellement apparenté à l'Aβ mais dépourvu d'activité SOD et isoler les peptides non liés ; ou
(ii) lorsque l'activité SOD de l'Aβ est dépendante d'une liaison au cuivre, mettre en contact les peptides isolés à l'étape (i) avec un deuxième agent qui est structurellement apparenté à l'Aβ mais qui n'est pas adapté pour une liaison au cuivre, et isoler les peptides non liés ; ou
(ii) lorsque la toxicité de l'Aβ est dépendante d'une liaison au cuivre, mettre en contact les peptides isolés à l'étape (i) avec un deuxième agent qui est adapté pour se lier aux peptides isolés à l'étape (i) et qui ne se lie pas spécifiquement au site de liaison au cuivre de l'Aβ, et isoler les peptides non liés ;
où les peptides isolés à l'étape (ii) sont des peptides candidats pour le traitement de la maladie.

2. Procédé selon la revendication 1, dans lequel les étapes (i) et (ii) du procédé sont répétées afin d'augmenter la sélectivité du procédé.

3. Procédé selon la revendication 1, dans lequel l'Aβ contient un site de liaison au cuivre et/ou une activité SOD ou une partie de celle-ci possède une activité SOD et/ou un site de liaison au cuivre.

4. Procédé selon la revendication 3, dans lequel l'Aβ est une Aβ humaine.

5. Procédé selon la revendication 3, dans lequel l'Aβ est une Aβ mammalienne non humaine.

6. Procédé selon l'une quelconque des revendications 3 à 5, dans lequel le deuxième agent est une Aβ qui est dépourvue d'activité SOD et/ou d'un site de liaison au cuivre ou une partie de celle-ci qui est dépourvue d'activité SOD et/ou d'un site de liaison au cuivre.

7. Procédé selon la revendication 6, dans lequel le deuxième agent est une Aβ de rat ou une Aβ de souris.

8. Procédé selon la revendication 1 pour isoler des peptides candidats pour le traitement de la maladie d'Alzheimer, le procédé comprenant les étapes consistant à :
(i) mettre en contact une pluralité de peptides candidats avec une Aβ et isoler les peptides liés ; et
(ii) mettre en contact les peptides isolés à l'étape (i) avec un deuxième agent qui est adapté pour se lier aux peptides isolés à l'étape (i) et qui ne se lie pas spécifiquement au site de liaison au cuivre de l'Aβ, et isoler les peptides non liés ;
où les peptides isolés à l'étape (ii) sont des peptides candidats pour le traitement de la maladie d'Alzheimer.

9. Peptide consistant en une séquence sélectionnée dans le groupe de séquences :
(i) Met-Thr-Met-Pro-Thr-Met ;
(ii) Pro-Leu-Pro-Gln-Met-Leu ; et
(iii) Thr-Asn-Pro-Asn-Arg-Arg-Asn-Arg-Thr-Pro-Gln-Met-Leu-Lys-Arg ;
ou un variant fonctionnel de celles-ci qui est au moins 80 % identique à la séquence décrite et qui conserve son aptitude à se lier à un agent causal de la maladie d'Alzheimer, ledit agent causal ayant une activité SOD, et inhibe l'aptitude de liaison à SOD et/ou à un ion métallique de l'agent causal.

10. Peptide selon la revendication 9 qui se lie au niveau ou à proximité d'un site de liaison au cuivre de l'Aβ et empêche physiquement la liaison du cuivre.

11. Peptide selon la revendication 9, où le peptide se lie au niveau ou à proximité des acides aminés 5 à 14 de l'Aβ humaine, ou aux acides aminés 8 à 14 de l'Aβ humaine, ou à l'acide aminé 13 de l'Aβ humaine.

12. Peptide selon la revendication 9 qui se lie à l'Aβ et perturbe la conformation (ou la structure tridimensionnelle) du site de liaison au cuivre afin de réduire ou de totalement éliminer son aptitude à se lier au cuivre et/ou son activité SOD.

13. Peptide selon la revendication 12, qui se lie à et perturbe la conformation des acides aminés 5 à 14 de l'Aβ humaine, ou des acides aminés 8 à 14 de l'Aβ humaine, ou de l'acide aminé 13 de l'Aβ humaine.

14. Peptide selon la revendication 9, qui se lie à l'Aβ et réduit ou élimine totalement son activité SOD tout en permettant toujours à l'Aβ de se lier au cuivre.

15. Polynucléotide codant pour l'un quelconque des peptides selon les revendications 9 à 14.

16. Peptide selon l'une quelconque des revendications 9 à 14, destiné à être utilisé dans un procédé thérapeutique dans le traitement de la maladie d'Alzheimer, de préférence afin de réduire ou d'éliminer une activité SOD ou d'inhiber l'aptitude de liaison au cuivre d'un agent causal de la maladie d'Alzheimer, le procédé consistant à mettre en contact l'agent causal avec ledit peptide de sorte que le peptide se lie à l'agent causal d'une manière qui au moins réduit son activité SOD et/ou de liaison au cuivre, ou afin de déstabiliser des formes multimériques de l'Aβ, le procédé consistant à mettre en contact l'Aβ multimérique avec ledit peptide de sorte que le peptide se lie à l'Aβ multimérique d'une manière qui déstabilise l'Aβ multimérique, ou afin de limier ou d'empêcher l'agrégation de l'Aβ, le procédé consistant à mettre en contact l'Aβ avec ledit peptide de sorte que le peptide se lie à l'Aβ et empêche ou limite son agrégation.

17. Procédé pour isoler des peptides candidats pour le traitement de la maladie d'Alzheimer, le procédé comprenant les étapes consistant à :
(i) mettre en contact une pluralité de peptides candidats avec un premier agent qui se lie à des peptides qui se lient spécifiquement au site de liaison au zinc de l'Aβ, et isoler les peptides liés ; et
(ii) mettre en contact les peptides isolés à l'étape (i) avec un deuxième agent qui se lie aux peptides isolés à l'étape (i) mais ne se lie pas spécifiquement au site de liaison au zinc de l'Aβ, et isoler les peptides non liés ;
où les peptides isolés à l'étape (ii) sont des peptides candidats pour le traitement de la maladie d'Alzheimer.
